# EUROPÄISCHE PATENTSCHRIFT

(11) **EP 2 842 481 B1**
(45) Veröffentlichungstag und Bekanntmachung des Hinweises auf die Patenterteilung: **12.07.2017**
(21) Anmeldenummer: 14186223.5
(22) Anmeldetag: 23.05.2008
(51) Int. Cl.: A61B 5/00, A61K 9/50, G01N 21/64

(54) **Hydrogel-Implantat für Sensorik von Metaboliten in Körpergewebe**
Hydrogel implant for sensors for metabolites in body tissue
Implant en hydrogel pour technique sensorielle de métabolites dans des tissus corporels

(30) Priorität: 24.05.2007 DE 102007024642; 25.05.2007 US 924669 P
(43) Veröffentlichungstag der Anmeldung: 04.03.2015
(62) Teilanmeldung aus: 08759965.0
(73) Patentinhaber: EyeSense AG, 4051 Basel (CH)
(72) Erfinder: Müller, Achim, 63762 Großostheim (DE); Herbrechtsmeier, Peter, 61462 Königstein (DE); Knuth, Monika, 63773 Goldbach (DE); Nikolaus, Katharina, 63739 Aschaffenburg (DE)
(74) Vertreter: Herzog, Fiesser & Partner Patentanwälte PartG mbB

(56) Entgegenhaltungen:
- WO-A-01/13783
- US-A1- 2004 180 391
- US-A1- 2005 095 174
- US-A1- 2007 105 176
- HUIGUANG ZHU ET AL: "Combined Physical and Chemical Immobilization of Glucose Oxidase in Alginate Microspheres Improves Stability of Encapsulation and Activity", BIOCONJUGATE CHEMISTRY, Bd. 16, Nr. 6, 1. November 2005 (2005-11-01), XP055161595, ISSN: 1043-1802, DOI: 10.1021/bc050171z

## Beschreibung

### Gebiet der Erfindung

Die Erfindung betrifft Hydrogelformkörper, die so aufgebaut sind, dass ein zu bestimmender Analyt in der wässrigen Phase eines Hydrogel-Netzwerks frei diffundieren kann, die chemischen bzw. biochemischen Sensorkomponenten aber im Netzwerk immobilisiert sind. Die äußere Form und die mechanischen Eigenschaften des Hydrogelformkörpers sind für die Implantation und den Implantationsort optimiert. Derartige Hydrogelformkörper können beispielsweise eingesetzt werden, um Analyten, insbesondere bestimmte Metaboliten, in einem Körpergewebe, insbesondere einer Körperflüssigkeit, zu detektieren. Insbesondere kann es sich bei dem Körpergewebe um ein Körpergewebe eines Auges handeln und bei der Körperflüssigkeit um eine Augenflüssigkeit (z.B. Kammerwasser, Tränenflüssigkeit oder interstitielle Flüssigkeit). Auch für andere Gewebearten und/oder Arten von Körperflüssigkeiten ist der vorgeschlagene Hydrogelformkörper jedoch grundsätzlich einsetzbar.

Die Detektion des mindestens einen zu bestimmenden Analyten kann von einer rein qualitativen Detektion bis hin zu einer quantitativen Detektion reichen. Derartige Detektionsverfahren können beispielsweise zur Bestimmung einer Glukosekonzentration in dem Körpergewebe, beispielsweise in der Augenflüssigkeit, genutzt werden. Aus dieser Analyt- bzw. Glucosekonzentration kann dann anschließend unter Bezugnahme auf bekannte Korrelationen beispielsweise auf eine Konzentration des Analyten, insbesondere der Glucose, in anderen Körperflüssigkeiten, beispielsweise in Blut geschlossen werden. Neben Glucose ist die vorliegende Erfindung, alternativ oder zusätzlich, auch auf andere Arten von Analyten anwendbar.

### Stand der Technik

Herkömmliche Systeme zur Bestimmung von Analyt- bzw. Metabolitkonzentrationen, insbesondere der Blutglukosekonzentration, basieren in der Regel darauf, dass der Patient oder ein Arzt, beispielsweise mittels eines geeigneten Lanzettensystems, einen Hautbereich perforiert und dadurch eine Blutprobe generiert. Diese Probe wird anschließend mittels geeigneter Messverfahren, beispielsweise optischer und/oder elektrochemischer Messverfahren, auf ihren Analytgehalt analysiert. Neben einem Nachweis in Blut kann auch ein Nachweis in anderen Körperflüssigkeiten, wie beispielsweise in Urin erfolgen.

Um die mit der häufigen Generierung von Blutproben verbundenen Unannehmlichkeiten der Patienten zu verringern, wurden verschiedene nicht-invasive oder minimal-invasive Technologien zur Messung von Analytkonzentrationen entwickelt. Im Folgenden wird dabei ohne Beschränkung des Schutzumfangs der Erfindung auf die Bestimmung der Blutglukosekonzentrationen eingegangen, wobei naturgemäß auch andere Arten von Analyten bzw. Metaboliten nachweisbar sind.

Eine Technologie der Blutglukosekonzentrationsbestimmung basiert auf der Messung von Glukose in Körpergewebe und Körperflüssigkeiten, insbesondere in Augenflüssigkeiten, wie beispielsweise Tränenflüssigkeit, Kammerwasser oder interstitieller Flüssigkeit. So ist beispielsweise in WO 01/13783 ein Okularsensor für Glukose beschrieben, welcher als Augenlinse ausgestaltet ist. Der Okularsensor umfasst einen Glukoserezeptor, welcher mit einem ersten Fluoreszenzlabel markiert ist, und einen Glukose-Konkurrenten, welcher mit einem zweiten Fluoreszenzlabel ("Donor") markiert ist. Die beiden Fluoreszenzlabel sind derart ausgewählt, dass, wenn der Konkurrent an den Rezeptor gebunden ist, die Fluoreszenz des zweiten Fluoreszenzlabels aufgrund eines resonanten Fluoreszenz-Energietransfers gelöscht wird (Quenching). Durch Überwachen der Veränderung der Fluoreszenzintensität bei einer Wellenlänge um das Fluoreszenzmaximum des quenchbaren Fluoreszenzlabels kann der Anteil des Fluoreszenz-markierten Konkurrenten gemessen werden, welcher durch die Glukose verdrängt worden ist. Auf diese Weise kann die Glukosekonzentration in der Augenflüssigkeit bestimmt werden. Diese Messung kann wiederum genutzt werden, um daraus auf die Blutglukosekonzentration zu schließen. Auch andere Arten von Nachweisen sind denkbar und dem Fachmann geläufig, beispielsweise ein Fluoreszenznachweis des ersten Fluoreszenzlabels.

Auch WO 02/087429 beschreibt ein Fluoreszenz-Photometer, mittels dessen Blutglukosekonzentrationen durch Messung der Glukosekonzentration in einer Augenflüssigkeit bestimmt werden können. Die dargestellte Vorrichtung ist in der Lage, gleichzeitig zwei Fluoreszenzintensitäten bei zwei verschiedenen Wellenlängen zu messen.

Die zitierten Druckschriften aus dem Stand der Technik stellen nur einige Ausführungsbeispiele dafür dar, wie Analyten durch geeignete Sensoren in einem Implantat, beispielsweise einem Augenimplantat, erfasst und in ihrer Konzentration bestimmt werden können. Ein zentraler Aspekt stellt jedoch in den meisten Fällen die Ausgestaltung des Implantats, insbesondere des Augenimplantats, selbst dar, welches für die Analytik zahlreichen Anforderungen und Randbedingungen genügen muss. Als geeignetes Matrixmaterial für derartige Implantate haben sich insbesondere Hydrogele erwiesen. Hydrogele sind wasserenthaltende, jedoch zumindest weitgehend wasserunlösliche Polymere, deren Moleküle chemisch, z. B. durch kovalente oder ionische Bindungen, oder physikalisch, z. B. durch Verschlaufen der Polymerketten, zu einem dreidimensionalen Netzwerk verknüpft sind. Hydrogele weisen in der Regel hydrophile Polymerkomponenten auf, welche bewirken, dass die Hydrogele in Wasser unter beträchtlicher Volumenzunahme aufquellen, wobei jedoch ihr stofflicher Zusammenhalt zumindest weitgehend erhalten bleibt. Hydrogele weisen eine hohe Biokompatibilität auf und haben zumeist gewebeähnliche mechanische Eigenschaften.

Hydrogelformkörper mit bestimmten Zusatzstoffen, die im Hydrogel-Netzwerk eingebettet sind, sind aus dem Stand der Technik bekannt, wobei unter einem Hydrogel-Netzwerk ein wasserenthaltendes Netzwerk verstanden wird, das aus einem Polymer aufgebaut ist, das entweder an sich wasserunlöslich ist oder durch geeignete Maßnahmen wasserunlöslich gemacht wurde. Zu solchen geeigneten Maßnahmen kann sich insbesondere die Erzeugung von kovalenten oder ionischen Bindungen zwischen den Polymerbausteinen des Netzwerks gehören; auch physikalische Maßnahmen wie Verschlaufungen der Polymerbausteine sind bekannt.

Zu den Hydrogelformkörpern, die im Stand der Technik beschrieben werden, zählen z. B. Implantate für das Auge, die entweder von außen auf die Oberfläche des Auges aufgebracht (z. B. Kontaktlinsen) oder in eine Schicht beziehungsweise Kammer des Auges implantiert (z. B. Intraokularlinsen) werden. Beispiele dafür sind die in den nachfolgend aufgeführten Patentdokumenten beschriebenen Formkörper. Bei den aus der US2005/0095174 bekannten Implantaten sind Sensorpartikel in einer Hydrogelmatrix immobilisiert, wobei die Sensorpartikel eine Sensormatrix mit einem Sensormatrixmaterial und mindestens ein Sensormaterial aufweisen. Das ophthalmische Implantat zur Kontrolle des Grauen Stars aus der US 5,127,901 wird zwischen die Sclera (Lederhaut) und die Konjunktiva (Bindehaut) eingebracht und weist eine dafür geeignete Form auf.

Die Implantate der US 5,300,114 oder US 5,476,511 eröffnen die Möglichkeit, dass unterhalb der Konjunktiva medizinisch aktive Stoffe wirksam sein können. Ethylen-VinylacetatCopolymere werden als besonders geeignetes Polymer für das Implantat angesehen, das auch eine geeignete Diffusionsbarriere für den gezielt freizugebenden Wirkstoff darstellt, der sich z. B. in einer inneren Matrix aus diesem Polymer befindet. Auch die Matrix mit dem Wirkstoff umhüllende Membran ist aus diesem Polymer aufgebaut. Zusätzlich enthalten diese Implantate einen Zusatzstoff, der den Verbrauch des Wirkstoffs anzeigt. Außerdem können diese Implantate auch Beschichtungen oder Abschnitte an bestimmten Stellen des Formkörpers aufweisen, die gerade nicht durchlässig - auch nicht zeitweilig - für den Wirkstoff sind, falls dies an bestimmten Stellen des Auges so erwünscht ist.

Die Implantate der US 6,416,777 und US 6,986,900 werden so ins Auge eingebracht, dass der medizinisch aktive Wirkstoff oberhalb der Macula (gelber Fleck der Netzhaut) angeordnet ist und das Implantat sich außerhalb der Sclera befindet. Ihre Geometrien weisen F-, C- oder L-Form auf. Das den Wirkstoff enthaltende Innere kann z. B. Tablettenform haben und das Polymere kann - je nach Anwendungsziel - für den Wirkstoff mehr oder weniger durchlässig sein. Das Polymer soll biokompatibel und nicht biologisch abbaubar sein. Acrylate und Silikone werden als bevorzugt aufgeführt. In einer Variante ist der Wirkstoff in einer Flüssigkeit gelöst, so dass für eine gezielte Abgabe aus dem Implantat gesorgt werden muss.

Die an solche Formkörper mit einem medizinisch aktiven Wirkstoff gestellten Anforderungen sind aber nicht ohne weiteres auf Formkörper übertragbar, in die Analyten eindringen und dort untersucht werden sollen. In letzterem Fall, bei welchem durch den Hydrogel-Formkörper Analyten nachgewiesen werden sollen, sind sogar oftmals diametral entgegen-gesetzte Anforderungen im Vergleich zu Wirkstoff-Implantaten zu stellen, da das bzw. die Sensormaterialien ja gerade nicht oder nur geringfügig im Implantat diffundieren sollen, sondern ortsgebunden im Implantat verbleiben sollen. Andererseits soll es dem nachzuweisenden Analyten ermöglicht werden, nahezu ungehindert und schnell zum Ort des Nachweises im Implantat zu diffundieren, damit die Analytkonzentration zeitnah erfasst werden kann. Dies stellt eine wesentliche Voraussetzung dafür dar, dass ggf. medizinische Gegenmaßnahmen ergriffen werden können, wie beispielsweise eine entsprechende Medikation von Insulin.

### Aufgabe der Erfindung

Aufgabe der vorliegenden Erfindung ist es daher, einen Hydrogelformkörper bereitzustellen, welcher den Nachweis eines oder mehrerer Analyten in einer Körperflüssigkeit, beispielsweise einer Augenflüssigkeit, ermöglicht und welcher die Nachteile bekannter Hydrogelformkörper zumindest weitgehend vermeidet. Insbesondere soll ein Hydrogelformkörper bereitgestellt werden, dessen äußere Form und dessen weiterer Aufbau es ermöglichen, dass sich im Hydrogel neben einem zu bestimmenden Analyten (z. B. Glukose), mindestens eine Sensorkomponente und gegebenenfalls noch mindestens eine Referenzkomponente befinden.

### Beschreibung der Erfindung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Ein Grundgedanke der vorliegenden Erfindung besteht darin, eine Immobilisierung einer Sensorkomponente in dem Implantat durch eine Verkapselung der Komponenten in Mikro-oder Nanopartikeln vorzunehmen, die in einer Hydrogelmatrix verteilt, insbesondere dispergiert, sind. Besonders bevorzugt ist dabei eine zumindest im Wesentlichen homogene Verteilung.

Es wird daher ein Implantat zum Nachweis mindestens eines Analyten in einer Körperflüssigkeit, insbesondere einer Augenflüssigkeit, vorgeschlagen, welches eingerichtet ist, um in ein Körpergewebe eines Patienten, insbesondere eine Gewebeschicht und/oder eine Kammer eines Auges des Patienten, implantiert zu werden. Der Begriff eines Patienten umfasst dabei allgemein Lebewesen, insbesondere Menschen, wobei der Begriff nicht notwendigerweise eine Krankheit impliziert. So können auch beispielsweise Messungen an gesunden Menschen oder Tieren vorgenommen werden, um eine Metabolitkonzentration zu messen, um gegebenenfalls rechtzeitig Krankheiten erkennen zu können. Der Begriff Implantat soll jedoch auch den Fall umfassen, dass keine Implantation im eigentlichen Sinne vorgenommen wird, d.h. eine Einbringung in ein Gewebe eines Patienten, sondern soll auch ein einfaches Aufbringen auf ein derartiges Gewebe umfassen, also ein Aufbringen ohne die Notwendigkeit eines chirurgischen Eingriffes, also beispielsweise eine Kontaktlinse und/oder ein Inlay, welches beispielsweise unter einem Augenlid eines Patienten platziert werden kann.

Das Implantat weist eine Hydrogelmatrix mit mindestens einem Hydrogel auf, wobei das Implantat weiterhin in der Hydrogelmatrix dispergierte Sensorpartikel aufweist, wobei die Sensorpartikel mindestens eine Sensormatrix mit einem Sensormatrixmaterial (122) und mindestens ein Sensormaterial aufweisen.

Die Sensorpartikel sind vorzugsweise als Mikro- oder Nanopartikel ausgestaltet, vorzugsweise mit einem Partikeldurchmesser im Bereich weniger Mikrometer (z.B. <100 Mikrometer, vorzugsweise <20 Mikrometer) bis hin zu einigen 100 Nanometern.

Die Mikro- oder Nanopartikel sind vorzugsweise permeabel gegenüber dem Analyten entweder aufgrund ihrer Struktur oder aufgrund einer semipermeablen Hülle. Das Innere der Partikel ist so gestaltet, dass die Sensorkomponenten eine optimale Aktivität aufweisen.

Das Sensormaterial ist derart ausgestaltet, dass dieses sensitiv auf den nachzuweisenden Analyten reagiert. Vorzugsweise ist diese Sensoreigenschaft spezifisch für den nachzuweisenden Analyten. Dabei können, wie aus dem oben beschriebenen Stand der Technik bekannt, verschiedene Nachweisprinzipien eingesetzt werden. Beispielsweise kann der Analyt mit dem Sensormaterial chemisch reagieren (z.B. eine kovalente Bindung, eine Komplexbindung oder eine ähnliche Verbindung eingehen), wobei diese Bindung beispielsweise durch Änderung von Fluoreszenzeigenschaften des Analyten und/oder des Sensormaterials und/oder der Sensormaterial-Analyt-Verbindung nachgewiesen werden kann. Auch lockerere Bindungen sind möglich, beispielsweise physikalische Bindungen und/oder Annäherungen von Sensormaterial und Analyt, welche beispielsweise wiederum spektroskopisch nachgewiesen werden können. In jedem Fall ist das Sensormaterial jedoch derart ausgestaltet, dass sich mindestens eine nachweisbare physikalische und/oder chemische Eigenschaft des Implantats ändert, wenn sich die Analytkonzentration in der Körperflüssigkeit, insbesondere der Augenflüssigkeit, ändert bzw. wenn Analyt in der Körperflüssigkeit vorhanden ist.

Ein wesentlicher Aspekt und Vorteil der Erfindung ist die Tatsache, dass die Eigenschaften von Hydrogelmatrix und Sensorpartikeln getrennt optimiert werden können. So benötigt man Implantate mit einer guten mechanischen Festigkeit, die bei Hydrogelen im Wesentlichen durch höhere Netzwerkdichte und relativ geringen Wassergehalt erzielt werden kann.

Werden jetzt aber für das Sensormaterial beispielsweise relativ große Biomoleküle wie Con A (104 kD), Glukoseoxidase (63 kD), Glukosedehydrogenase, Hexokinase oder Glucose Galactose binding protein (GGBP) verwendet, deren Funktionalität vom Vorliegen der nativen Konfiguration sowie von der Beweglichkeit der Biomoleküle abhängt, so wirken sich allerdings geringe Wassergehalte und dichte Netzwerke ungünstig auf die Aktivität und Beweglichkeit der Proteine aus. In den Mikropartikeln können die Umgebungsbedingungen für solche Proteine und/oder andere Sensorkomponenten unabhängig von den Anforderungen an das Implantat optimiert werden. Weiterhin kann das Sensormaterial auch ein Protein und/oder ein funktionell äquivalentes Fragment umfassen, Mutanten von Hexokinase und/oder GGBP und/oder Borsäureesterderivate.

So können beispielsweise Hydrogele auch für die Mikropartikel bzw. Sensorpartikel verwendet werden, deren Wassergehalt über 90% beträgt. Da in solchen Fällen die Proteine wegen der geringen Netzwerkdichte teilweise aus den Partikeln heraus diffundieren könnten, werden die Sensorpartikel vorzugsweise mit einer semipermeablen Schicht beschichtet.

Dies können "klassische" LBL ("layer-by-layer")-Schichten sein, es können aber auch vernetzte Proteine, Polysaccharide oder andere Polymere eingesetzt werden, die eine zweite, dichtere Hydrogelschicht um das Innere des Partikels bilden. Der Begriff LBL bezieht sich dabei auch auf die nacheinander folgende Abscheidung von entgegengesetzt geladenen Polyelektrolyten. Man kann zum Beispiel einen Sensorpartikel zunächst mit einem negativ oder positiv geladenen Polyelektrolyten beschichten und anschließend mit einem entsprechend entgegengesetzt geladenen Polyelektrolyten. Diesen Vorgang kann man wiederholen, bis die gewünschte Schichtdicke und Permeabilität erzielt wird. Es gibt auch Varianten, bei denen teilweise ungeladene Polymerschichten zwischen zwei entgegengesetzt geladenen Schichten eingebracht werden. Alternativ kann man die "LBL" Schicht auch nicht schrittweise sondern durch einen Schritt aufbauen, indem in der Beschichtungslösung Komplexe aus den beiden entgegengesetzt geladenen Polyelektrolyten gebildet werden, die sich unter bestimmten Bedingungen auf der Oberfläche der Partikel abscheiden. Sind die Sensorkomponenten sehr groß oder ist die die Mikropartikel umhüllende Hydrogelmatrix besonders dicht, so können auch Mikropartikel ohne Membran verwendet werden.

Geeignete Lösungen für solche speziellen Sensorpartikel, insbesondere in dem Aufbau der LBL-Schichten, werden beispielsweise in den folgenden Patentdokumenten offenbart: WO 2005/089727, WO 2004/014540, WO 02/017888, WO 00/077281, WO 00/003797, EP-A 1 116 516, WO 99/047252, WO 99/047253, US 6,451,871, US 6,896,926, US 7,022,379 und US 6,926,965.

Geeignete Materialien für Sensorpartikel sind z.B. ionisch vernetzte Alginate und Mischungen von Alginaten und Polysacchariden oder Polysaccharidderivaten wie Carboxymethylcellulose oder aber auch synthetische Polymere bzw. Copolymere wie Polyhydroxyethylmethacrylat (P-HEMA), Polyacrylamide und Copolymere aus Acrylsäure und/oder Acrylsäure- und Methacrylsäurederivate wie Dimethylacrylamid, Hydroxyethyacrylat, Methacrylsäure. Grundsätzlich sind alle Polymere denkbar, die wasserlöslich und vernetzt oder vernetzbar sind. Es ist auch möglich, für die Sensorpartikel das gleiche Polymer wie für die Hydrogelmatrix zu benutzen, wobei sich im Allgemeinen jedoch die Polymere in ihrem Vernetzungsgrad unterscheiden sollten. Ein Beispiel hierfür sind Polyvinylalkohole mit unterschiedlichen Mengen funktioneller, vernetzbarer Gruppen.

Geeignete Hydrogele für die Sensorpartikel und/oder auch für die Hydrogelmatrix werden z. B. in den nachfolgenden Patentdokumenten offenbart: EP-B 0 641 806, EP-B 0 790 258, EP-B 0 807 265 und EP 0 637 490.

Neben Sensorpartikeln mit Mikro- oder Nanopartikeln, die die Sensormaterialien bzw. Sensorkomponenten enthalten, weist das Implantat weiterhin mindestens eine zumindest weitgehend Analyt-invariante Referenzkomponente auf. Die Referenzkomponente kann insbesondere mindestens eine lumineszierende Komponente, insbesondere eine Fluoreszenzkomponente, aufweisen. Die Lumineszenzeigenschaften der luminszierenden Komponente sollen zumindest weitgehend Analyt-invariant sein.

Die Referenzkomponente wird mittels Referenzpartikeln in das Implantat eingebracht. So können Referenzpartikel in die Hydrogelmatrix eingebettet werden, die eine oder mehrere Referenzkomponenten enthalten. Weiterhin kann ein Referenzmatrixmaterial enthalten sein. Wiederum können diese Referenzpartikel vorzugsweise Mikro- oder Nanopartikel aufweisen, vorzugsweise mit einem Partikeldurchmesser im Bereich weniger Mikrometer (z.B. <100 Mikrometer, vorzugsweise <10 Mikrometer) bis hin zu einigen 100 Nanometern.

Grundsätzlich kann für das Referenzmatrixmaterial das oben bezüglich der Hydrogelmatrix Gesagte entsprechend gelten. Insbesondere können für das Referenzmatrixmaterial ebenfalls eine oder mehrere der oben beschrieben Materialien eingesetzt werden. Auch die Verwendung einer Hülle um die Referenzpartikel ist wiederum möglich, wobei bezüglich der Materialien und weiteren Eigenschaften ebenfalls wiederum auf das oben zur Hülle der Sensorpartikel Gesagte analog verwiesen werden kann. Die Sensor- und/oder Referenzpartikel sollten dabei relativ klein bezogen auf die Dicke des Hydrogelformkörpers sein, damit eine homogene Verteilung im Hydrogel bzw. Referenzmatrixmaterial möglich ist. Der Durchmesser sollte vorzugsweise nicht größer als ca. 10 % der Dicke des Hydrogels bzw. des Hydrogelformkörpers sein.

Die Referenzkomponenten können z. B. Fluoreszenzfarbstoffe oder hochmolekulare Derivate von Fluoreszenzfarbstoffen sein oder umfassen, die entweder auf der Oberfläche des Hydrogels, der Sensorpartikel und/oder der Referenzpartikel oder in der Matrix (Matrixmaterial) der Referenz- oder Sensorpartikel chemisch oder physikalisch gebunden sind.

Vorzugsweise sind die Referenzkomponenten zumindest weitgehend Analyt-invariant, d.h. ändern ihre nachweisbaren physikalischen und/oder chemischen Eigenschaften (z.B. wiederum Fluoreszenz- und/oder Lumineszenzeigenschaften) im Wesentlichen auch bei Anwesenheit des nachzuweisenden Analyten nicht oder nur unwesentlich (z.B. um nicht mehr als 5%, vorzugsweise weniger).

Zur oberflächlichen Anbindung der Farbstoffe können kovalente Bindungen dienen oder aber auch starke Komplexbindungen wie Biotin-Avidin. In diesen Fällen werden funktionelle Gruppen an der Oberfläche der Partikel mit funktionellen Gruppen am Farbstoffmolekül umgesetzt. Entsprechende Synthesevorschriften zur Kopplung von z.B. Amino-Gruppen, Thiol-Gruppen und Carboxy-Gruppen sind literaturbekannt. Auch können die Farbstoffe in LBL-Schichten oder anderen Schichten, die auf inerten Partikeln aufgebracht werden, eingebettet sein. In diesen Fällen kann der Farbstoff entweder aufgrund seiner z.B. Ladungseigenschaften zusammen mit den Polyelektrolyten abgeschieden werden, oder der Farbstoff ist direkt an einen der Polyelektrolyten kovalent gebunden.

Zur Bindung in den Partikeln können die Referenzkomponenten (im Folgenden ohne Beschränkung der allgemeinen Ausgestaltungsmöglichkeiten auch einfach "Farbstoffe" oder "Farbstoffmolekül" oder "Farbstoffgruppe" genannt) zum Beispiel direkt mit Monomeren polymerisiert werden und als Partikel gestaltet werden. In diesem Fall ist das durch die Polymerisation der Monomere entstehende Netzwerk vorzugsweise so engmaschig, dass das Farbstoffmolekül nicht mehr hinaus diffundieren kann. Eine solche physikalische Immobilisierung kann auch durch Quellung der Partikel in geeigneten Lösungsmitteln und Inkubation der gequollenen Partikel in einer Farbstofflösung erreicht werden. Dabei macht man sich die Tatsache zunutze, dass das Netzwerk in guten Lösungsmitteln seine Porengröße vergrößert (z.B. Polystyrol in Toluol) und nach Eindiffundieren der Farbstoffmoleküle im Anwendungslösungsmittel (Wasser oder physiologischer Lösung) die Porengröße wieder verringert. Dies ist besonders bei empfindlichen Farbstoffen von Vorteil, da so für den Farbstoff die Bedingungen der Polymerisation umgangen werden.

Eine andere Variante besteht darin, dass das Farbstoffmolekül selbst polymerisierbare funktionelle Gruppen enthält und zusammen mit dem Monomer copolymerisiert wird. Die Referenzpartikel zeichnen sich dadurch aus, dass sich ihr Messparameter z.B. Fluoreszenz nicht mit der Konzentration des Analyten ändert.

Das Implantat kann insbesondere einen Hydrogelformkörper aufweisen. Der Hydrogelformkörper selbst wird dann vorzugsweise aus einem wasserlöslichen vernetzbaren Präpolymer und den Sensor- und Referenzpartikeln hergestellt. Dabei werden die Partikel in einer wässrigen Lösung der Präpolymeren homogen dispergiert und die wässrige Dispersion anschließend vernetzt (radikalisch z.B. photochemisch oder thermisch oder in 2+2 Cycloaddition).

Der Formkörper weist bevorzugt einen maximalen Durchmesser von 10 mm und ein Oberflächen zu Volumen-Verhältnis von mindestens 8 auf. Diese Weiterbildung der Erfindung bewirkt, dass die Ansprechgeschwindigkeit des Implantats auf Änderungen der Analytkonzentration in der Augenflüssigkeit typischerweise einen Wert von wenigen Minuten, vorzugsweise von nicht mehr als 3-4 Minuten, nicht übersteigt. Der Formkörper muss nicht zwangsläufig eine runde Scheibe sein. Vielmehr sind beliebige Formen möglich, solange der die Form umschreibende Kreis nicht größer 10 mm ist.

Der Rand des Formkörpers kann im Wesentlichen rechtwinklig sein, wobei jedoch unter "im Wesentlichen" auch Abweichungen von bis zu 60°, vorzugsweise jedoch von nicht mehr als 20° und besonders von nicht mehr als 5° toleriert werden können. Die Dicke des Formkörpers nimmt bevorzugt zum Rand hin ab. Der Rand hat einen bevorzugten Winkel von 0° bis 60°. Die Kanten können bevorzugt abgerundet sein. Der Formkörper kann planar oder gewölbt sein. Die Wölbung hat bevorzugt einen Krümmungsradius von 14 mm bis 8 mm. Der Krümmungsradius der Wölbung soll insbesondere nicht kleiner 8 mm sein.

Zusammengefasst kann der Hydrogelformkörper, welchen das Implantat aufweisen kann, insbesondere über die folgenden Eigenschaften verfügen:
- Der Hydrogelformkörper kann eine im Wesentlichen flache, runde Gestalt aufweisen, wobei der Durchmesser des Hydrogelformkörpers nicht größer sein kann als 10 mm.
- Der Hydrogelformkörper kann ein Oberflächen-zu-Volumen-Verhältnis von mindestens 5, vorzugsweise von mindestens 8 aufweisen.
- Der Hydrogelformkörper kann einen im Wesentlichen rechtwinkligen Rand aufweisen.
- Der Hydrogelformkörper kann eine Wölbung mit einem Krümmungsradius zwischen 5 mm und 20 mm, besonders bevorzugt zwischen 8 mm und 14 mm aufweisen.
- Der Hydrogelformkörper kann eine Dicke von nicht mehr als 250 Mikrometern aufweisen.
- Der Hydrogelformkörper kann im Randbereich eine Dicke von nicht mehr als 250 Mikrometern, vorzugsweise eine Dicke zwischen 15 Mikrometern und 250 Mikrometern aufweisen.
- Der Hydrogelformkörper kann einen abgerundeten Rand aufweisen, insbesondere einen abgerundeten Rand mit einem Gaußprofil.
- Der Hydrogelformkörper kann zumindest teilweise unter Verwendung eines Laserablationsverfahrens und/oder eines lithographischen Verfahrens und/oder eines Gussverfahrens hergestellt sein.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1A: eine Hydrogelmatrix eines Implantats mit Sensorpartikeln mit Membran;
- Figur 1B: eine Hydrogelmatrix eines Implantats mit Sensorpartikeln ohne Membran;
- Figur 2: einen Formkörper eines Implantats in verschiedenen Ansichten;
- Figur 3: eine Schnittdarstellung eines ersten Ausführungsbeispiels eines Formkörpers eines Implantats in einer Seitenansicht; und
- Figur 4: eine Schnittdarstellung eines zweiten Ausführungsbeispiels eines Formkörpers eines Implantats in einer Seitenansicht.

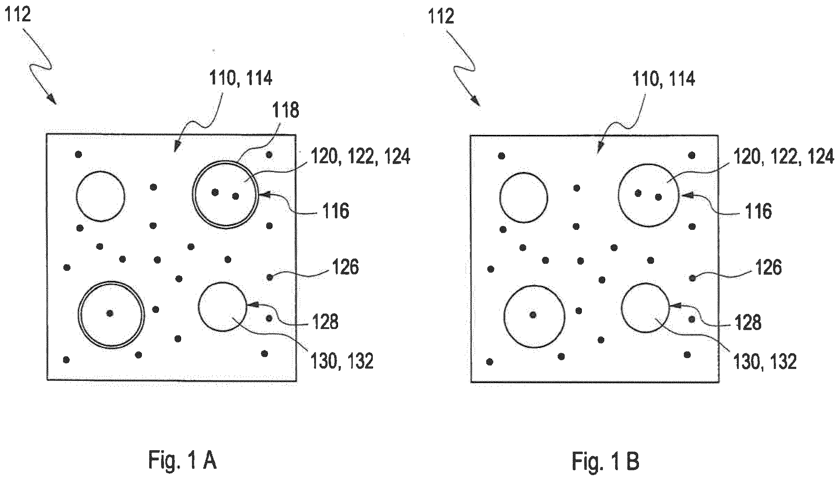

In den Figuren 1A und 1B ist jeweils eine Hydrogelmatrix 110 eines Implantats 112 (das Implantat ist nur symbolisch dargestellt) dargestellt. Im Folgenden wird speziell die Anwendung der Erfindung auf ein Augenimplantat erläutert, wobei jedoch, wie oben ausgeführt, die Erfindung grundsätzlich auch auf Implantate 112 für andere Arten von Körpergewebe einsetzbar ist. Die Hydrogelmatrix 110 des Implantats 112 weist jeweils als Grundkomponente ein Hydrogel 114 auf. Der Wassergehalt, die Netzwerkdichte und die Form der Hydrogelmatrix 110 können jeweils optimiert sein für die spezielle Implantationsanwendung.

In beiden Fällen sind in der Hydrogelmatrix 110 Sensorpartikel 116 verteilt. Die Ausführungsbeispiele in den Figuren 1A und 1B unterscheiden sich dadurch, dass in Figur 1A die Sensorpartikel 116 eine Membran 118 aufweisen, in dem Ausführungsbeispiel in Figur 1B hingegen nicht. Es sind jedoch auch Ausführungsformen denkbar, in denen sowohl Sensorpartikel 116 mit Membran 118 als auch solche ohne Membran nebeneinander vorliegen.

Die Sensorpartikel 116 weisen jeweils eine Sensormatrix 120 mit einem Sensormatrixmaterial 122 und einem in dem Sensormatrixmaterial aufgenommenen Sensormaterial 124 auf. Das Sensormaterial 124 ist sensitiv für einen Analyten 126, welcher in den Figuren 1A und 1B symbolisch mit der Bezugsziffer 126 bezeichnet ist und welcher durch die Hydrogelmatrix 110 und vorzugsweise auch durch die Sensormatrix 120 diffundieren kann.

Weiterhin sind in der Hydrogelmatrix 110 in den dargestellten Ausführungsbeispielen Referenzpartikel 128 verteilt. Diese weisen ein Referenzmatrixmaterial 130 und eine Referenzkomponente 132 auf, wobei die Referenzkomponente 132 in diesem Ausführungsbeispiel an der Oberfläche und/oder im Inneren des Referenzmatrixmaterials 130 physikalisch und/oder chemisch gebunden ist. Beispielsweise kann ein einpolymerisierter Fluoreszenzfarbstoff und/oder ein auf der Oberfläche des Referenzmatrixmaterials 130 und/oder des Referenzpartikels 128 angebrachter Fluoreszenzfarbstoff als Referenzkomponente 132 verwendet werden.

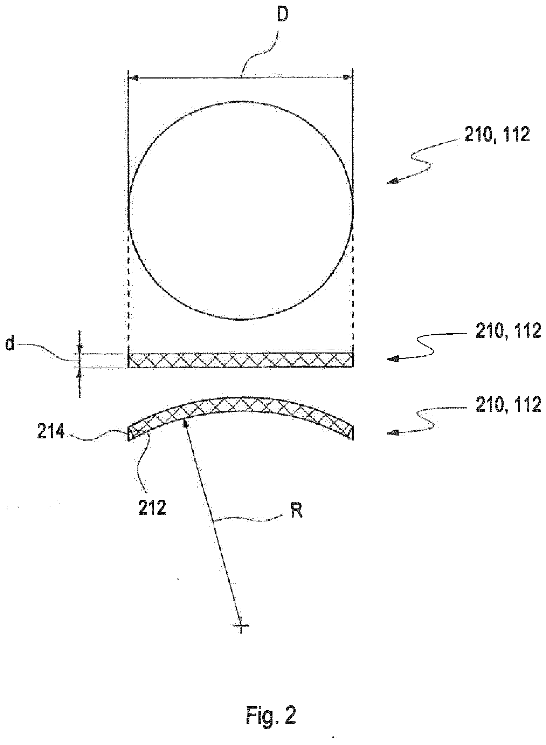

In Figur 2 ist ein Ausführungsbeispiel eines Formkörpers 210 eines Implantats 112 in verschiedenen Ansichten dargestellt. Die obere Ansicht zeigt eine Draufsicht, die mittlere Ansicht eine Schnittdarstellung von der Seite ohne Krümmung und die untere Ansicht eine Schnittdarstellung von der Seite mit einer Krümmung. Der Durchmesser D beträgt vorzugsweise nicht mehr als 10 mm, und die Dicke d vorzugsweise ca. 250 Mikrometer. Der Krümmungsradius R (unterste Darstellung) beträgt vorzugsweise zwischen 8 mm und 14 mm.

Weiterhin sind in der untersten Darstellung der Formkörpers 210 zwei mögliche Randformen überlagert dargestellt. Während die Randform 212 einen im Wesentlichen rechteckigen Rand zeigt, wie er beispielsweise mittels einer Gussform erzeugt werden kann, zeigt die Randform 214 eine spitz zulaufende Form. Hierbei sind vorzugsweise die Ränder der Randform 214 senkrecht zu einer Scheibenebene des Formkörpers 210. Ein derartiger Randverlauf 214 kann beispielsweise durch ein Lithographisches Herstellungsverfahren entstehen, bei welchem zur Aushärtung des Formkörpers 210 eine Bestrahlung senkrecht von oben erfolgt.

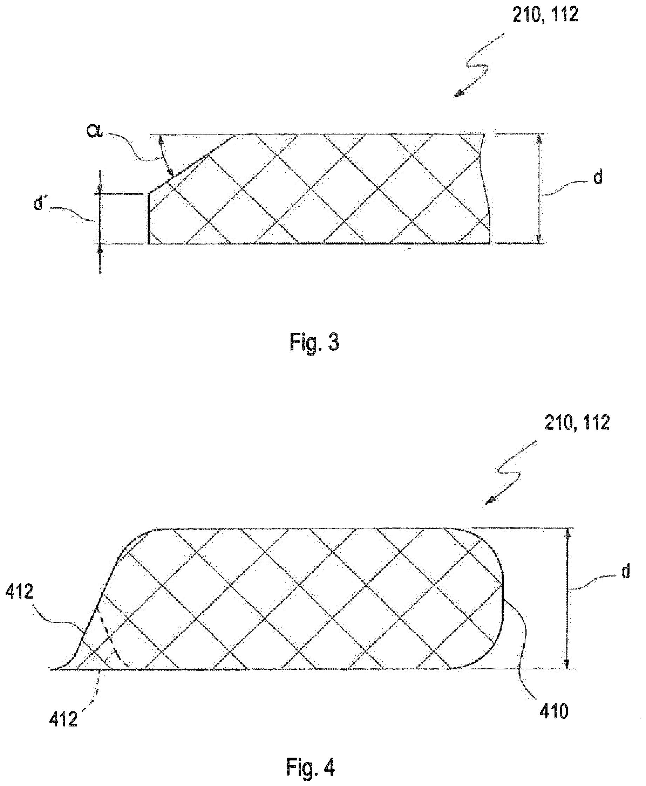

In den Figuren 3 und 4 sind weitere Ausführungsbeispiele von Randformen eines Formkörpers 210 dargestellt. So zeigt Figur 3 eine teilweise schräg verlaufende Randform. Dabei nimmt die Dicke des Formkörpers 210 von der Ausgangsdicke d zum Rand hin auf die Dicke d' ab. Während die Dicke d beispielsweise 250 Mikrometer betragen kann, kann die Randdicke d' beispielsweise 15 Mikrometer bis hin zu 250 Mikrometer betragen. Damit ergibt sich beispielsweise ein Randwinkel, welcher in Figur 3 mit α bezeichnet ist, von 0° bis hin zu 60°.

In Figur 4 sind wiederum zwei mögliche Randverläufe 410, 412 eines Formkörpers 210 überlagert, welche in weiteren Ausführungsbeispielen eingesetzt werden können. Hierbei ist mit der Bezugsziffer 410 eine Randgeometrie bezeichnet, welche (beispielsweise durch Verwendung einer entsprechenden Gussform) einen abgerundeten (z.B. kreisbogenförmigen oder elliptischen Verlauf) aufweist. Mit der Bezugsziffer 412 ist eine Randgeometrie bezeichnet, welche einen gekrümmten Verlauf aufweist, beispielsweise durch Verwendung eines Laserablationsverfahrens. Dieser gekrümmte Verlauf 412 kann einseitig vorgesehen sein (durchgezogene Linie 412) oder auch beidseitig (in Figur 4 gestrichelt dargestellt).

Die Form des Hydrogelformkörpers 210 kann beispielsweise durch eine entsprechende Gussform definiert werden. Die Gussform ist vorzugsweise so gefertigt, dass ein Schrumpfen oder Quellen bei der Aushärtung der Ausgangsformulierung für die Formgebung berücksichtigt wird. Die Gussform kann ganz oder teilweise aus einem Kunststoff wie Polypropylen (PP), Polymethylmethacrylat (PMMA), Polycarbonat (PC), Polyoxymethylen (POM) oder Polyetheresterketon (PEEK) oder aus Glas (UV-lichtdurchlässig) bestehen. Im Falle von geschlossenen Formen wird die Randgeometrie durch die geschlossene Gussform definiert. Im Falle von offenen Formen (Glasformen) kann der Rand photolithographisch durch UV-Vernetzung definiert werden oder durch die Oberflächenspannung zwischen Präpolymerlösung und Formmaterial.

Auch kann im Falle von offenen Formen oder größeren Formstücken der Rand durch Ausschneiden definiert werden. Bei einem mechanischen Ausschneiden ergibt sich eine weitgehend rechteckige Randgeometrie. Bei einem Ausschneiden mittels Laser kann bei Verwendung eines Gauß-Intensitätsprofils eine "abgerundete" Kante erzielt werden.

Nachfolgend wird die Herstellung eines Hydrogelformkörpers beispielhaft dargestellt.

### Beispiel 1 Herstellung von Alginathydrogelpartikeln für die Sensorkomponenten:

Alginsäure-Natriumsalz wird in deionisiertem Wasser bei 55°C unter Rühren gelöst. Die Alginatlösung wird mittels einer Zweistoffzerstäuberdüse (Firma Spraying Systems Co.) in ein mit Calciumchloridlösung befülltes Ultraschallbad versprüht, wo die Alginattropfen aushärten.

Die ausgehärteten Alginatpartikel werden über ein 30 µm Filtergewebe filtriert und das Filtrat durch Absitzen im Scheidetrichter aufkonzentriert. Anschließend werden die Alginatpartikel als 10%ige Lösung autoklaviert. Je nach gewünschtem Wassergehalt der Alginatpartikel kann die Konzentration der Alginatlösung zwischen 0,2% und 10% variiert werden. Durch entsprechende Wahl des Alginattyps (Molekulargewicht, Verhältnis Guluronsäure zu Manuronsäure) ist ein weiteres Feintuning der Netzwerkdichte möglich.

### Beispiel 2 Optionale Vorbeschichtung der Alginatpartikel:

Die Alginatpartikel werden abzentrifugiert und im Verhältnis 1:1 (w/v) mit Polyallylamin-Hydrochlorid in 10 mM Acetat-Puffer, pH 5,5 gemischt und 5 min inkubiert. Die Mischung wird zentrifugiert, der Überstand abgenommen und die Alginatpartikel zweimal im Verhältnis 1:2 (w/v) mit 10 mM Acetat-Puffer, pH 5,5 jeweils 2 min gewaschen und abzentrifugiert. Dieser Vorgang wird mit Polystyrolsulfonat in 10 mM Acetat-Puffer, pH 5,5 als zweite Schicht wiederholt. Der Vorgang wird so lange wiederholt, bis die gewünschte Anzahl Schichten aufgebracht wurde. Die Anzahl an Schichten sowie die Konzentration der Polyelektrolyte bestimmt die Dichte der Vorbeschichtung. Typische Konzentrationen sind zwischen 0,05% und 1%, typische Schichtzahlen zwischen 1 und 6.

### Beispiel 3 Befüllung der vorbeschichteten Alginatkugeln mit Sensorkomponenten Dextran und ConA:

Die (optional vorbeschichteten) Alginatpartikel werden abzentrifugiert, einmal mit deionisiertem Wasser gewaschen und wieder abzentrifugiert.

Die benötigte Menge Dextran wird abgewogen und in Wasser gelöst.

Auf 1 g abzentrifugiertes Alginatpartikel-Pellet wird 1 ml der Dextranlösung gegeben, durch Schütteln gemischt, im Ultraschallbad homogenisiert und über Nacht bei 2-8 °C inkubiert. Anschließend werden die Alginatkugeln abzentrifugiert und vom Überstand getrennt. Die aufgenommene Menge Dextran wird durch Differenzbildung der spezifischen Absorptionen von Überstand vor und nach Beladung errechnet. Typische Beladungen sind zwischen 0,01 und 10 mg Dextran pro g Alginatpartikel.

ConA wird in einer Konzentration von 5-15 mg/ml in TRIS-Puffer, pH 7,4 gelöst. Die benötigte Menge ConA wird auf das Dextran-befüllte Alginatpartikel-Pellet gegeben, durch Schütteln gemischt, im Ultraschallbad homogenisiert und über Nacht bei 2-8 °C inkubiert. Anschließend werden die Alginatkugeln abzentrifugiert und vom Überstand getrennt. Die aufgenommene Menge ConA wird durch Differenzbildung der proteinspezifischen Absorptionen von Überstand vor und nach Beladung errechnet.

### Beispiel 4 Beschichtung der Alginatpartikel:

Die beladenen (optional vorbeschichteten) Alginatkugeln werden im Verhältnis 1:1 (w/v) mit Polystyrolsulfonat in 10 mM Acetat-Puffer, pH 5,5 gemischt und 5 min inkubiert. Die Mischung wird zentrifugiert, der Überstand abgenommen und die Alginatkugeln zweimal im Verhältnis 1:2 (w/v) mit 10 mM Acetat-Puffer, pH 5,5 jeweils 2 min gewaschen und abzentrifugiert. Dieser Vorgang wird abwechselnd mit Polyallylamin-Hydrochlorid in 10 mM Acetat-Puffer, pH 5,5 und Polystyrolsulfonat in 10 mM Acetat-Puffer, pH 5,5 wiederholt, bis die gewünschte Anzahl Schichten aufgebracht sind. Die Anzahl an Schichten sowie die Konzentration der Polyelektrolyte bestimmt die Dichte der Vorbeschichtung. Typische Konzentrationen sind zwischen 0,05% und 1%, typische Schichtzahlen zwischen 10 und 60.

### Bsp. 5 Herstellung der Formulierung:

Eine 10%ige Referenzpartikel-Suspension wird im Ultraschallbad homogenisiert.

990 mg beschichtete Sensorpartikel werden mit 8,415 g einer 20 bis 40%igen Lösung von Acrylamidoacetaldehydo-1,3-acetal von Polyvinylalkohol durch Rühren gemischt. 495 µl 10%ige Referenzpartikel-Suspension werden zupipettiert und die Mischung im Ultraschallbad homogenisiert. Danach wird die Formulierung ca. 3 Stunden auf einem Rollenblock gerollt.

### Bsp. 6 Herstellung von Implantaten:

Die Formulierung wird in eine Spritze gefüllt und mittels einer Druckluft-betriebenen Dosiereinheit in einen Formkörper (weibliche Seite BK7 Glas, männliche Seite Quarzglas) dosiert. Der Formkörper wird geschlossen und unter UV-Licht (Hamamatzu Mercury-Xenon Lampe) für ca. 5 sec bestrahlt. Das vernetzte Implantat wird aus dem Formkörper entnommen, an Luft getrocknet und verpackt.

Es werden bereits Implantate mit 2 und 4 mm Durchmesser und einer Dicke von ca. 140 bis 250 µm hergestellt und ins menschliche Auge implantiert. Es kommen Implantate mit Krümmungsradien von 12 mm, 8,6 mm und planare Implantate zum Einsatz. Die Ränder werden durch Ausstanzen oder durch Formschluss definiert.

Es werden auch Ränder mit Phasen an der Ober- und Unterseite eingesetzt. Das Ausschneiden mittels Excimer Laser wird ebenfalls durchgeführt.

### Bezugszeichen

- 110: Hydrogelmatrix
- 112: Implantat
- 114: Hydrogel
- 116: Sensorpartikel
- 118: Membran
- 120: Sensormatrix
- 122: Sensormatrixmaterial
- 124: Sensormaterial
- 126: Analyt
- 128: Referenzpartikel
- 130: Referenzmatrixmaterial
- 132: Referenzkomponente

- 210: Formkörper
- 212: rechtwinklige Randform
- 214: spitz zulaufende Randform

- 410: runder Randverlauf
- 412: gekrümmter Randverlauf

## Patentansprüche

1. Implantat (110) zum Nachweis mindestens eines Analyten (126) in einer Körperflüssigkeit, insbesondere einer Augenflüssigkeit, wobei das Implantat (110) eingerichtet ist, um in ein Körpergewebe eines Patienten, insbesondere eine Gewebeschicht und/oder eine Kammer eines Auges des Patienten, implantiert zu werden, wobei das Implantat (110) eine Hydrogelmatrix (110) mit mindestens einem Hydrogel (114) aufweist, wobei das Implantat (110) weiterhin Sensorpartikel (116) aufweist, wobei die Sensorpartikel (116) mindestens eine Sensormatrix (120) mit einem Sensormatrixmaterial (122) und mindestens ein Sensormaterial (124) aufweisen, **dadurch gekennzeichnet, dass** das Implantat (110) weiterhin Referenzpartikel (128) aufweist, wobei die Referenzpartikel (128) ein Referenzmatrixmaterial (130) und eine daran gebundene und zumindest weitgehend Analyt-invariante Referenzkomponente (132) aufweisen, und dass die Sensorpartikel (116) und die Referenzpartikel (128) homogen in der Hydrogelmatrix (110) dispergiert sind.

2. Implantat (110) nach dem vorhergehenden Anspruch, wobei das Sensormaterial (124) mindestens eines der folgenden Materialien aufweist: Concanavalin A; Glukoseoxidase; Glukosedehydrogenase; Hexokinase; Glucose Galactose-bindendes Protein; ein Protein und/oder ein funktionell einem Protein äquivalentes Fragment; einen Mutanten von Hexokinase; einen Mutanten von Glucose Galactose-bindendem Protein; ein Borsäureesterderivat.

3. Implantat (110) nach einem der beiden vorhergehenden Ansprüche, wobei die Sensorpartikel (116) eine für den Analyten (126) zumindest teilweise permeable Hülle (118), insbesondere eine Membran, aufweisen.

4. Implantat (110) nach dem vorhergehenden Anspruch, wobei die Hülle (118) diffusionshemmende Eigenschaften für das mindestens eine Sensormaterial (124) aufweisen, insbesondere für das mindestens eine Sensormaterial (124) zumindest weitgehend undurchlässig ist.

5. Implantat (110) nach einem der beiden vorhergehenden Ansprüche, wobei die Hülle (118) mindestens eine der folgenden Materialien aufweist: ein vernetztes Protein; ein Polysaccharid; ein vernetztes Polysaccharid; ein Polymer, insbesondere ein Hydrogel mit einer die Dichte des Hydrogels der Hydrogelmatrix (110) übersteigenden Dichte; eine layer-by-layer-Schicht.

6. Implantat (110) nach einem der vorhergehenden Ansprüche, wobei das Sensormaterial (124) mindestens eines der folgenden Materialien aufweist:
- ein Molekül und/oder eine Gruppe, welche bei Anwesenheit eines Analyten (126) mindestens eine chemische oder physikalische Eigenschaft ändert, insbesondere eine Lumineszenzeigenschaft, insbesondere eine Fluoreszenzeigenschaft;
- ein Molekül und/oder eine Gruppe, wobei der Analyt (126) bei Annäherung und/oder Reaktion mit dem Molekül und/oder der Gruppe mindestens eine chemische oder physikalische Eigenschaft ändert, insbesondere eine Lumineszenzeigenschaft, insbesondere eine Fluoreszenzeigenschaft;
- ein Molekül und/oder eine Gruppe, wobei der Analyt (126) mit dem Molekül und/oder der Gruppe eine chemische oder physikalische Bindung eingeht, welche durch Änderung mindestens einer chemischen oder physikalischen Eigenschaft nachweisbar ist, insbesondere durch Änderung einer Lumineszenzeigenschaft, insbesondere durch Änderung einer Fluoreszenzeigenschaft.

7. Implantat (110) nach einem der vorhergehenden Ansprüche, wobei das mindestens eine Sensormatrixmaterial (122) und die Hydrogelmatrix (110) derart ausgestaltet sind, dass das mindestens eine Sensormaterial (124) in dem Sensormatrixmaterial (122) einen höheren Diffusionskoeffizienten aufweist als in der Hydrogelmatrix (HO), insbesondere als in dem mindestens einen Hydrogel (114) der Hydrogelmatrix (110), wobei das Sensormatrixmaterial (122) vorzugsweise eine geringere Dichte aufweist als die Hydrogelmatrix (110), insbesondere als das Hydrogel (114) der Hydrogelmatrix (110).

8. Implantat (110) nach einem der vorhergehenden Ansprüche, wobei das Sensormatrixmaterial (122) ein Hydrogel mit einem Wassergehalt aufweist, welcher den Wassergehalt des Hydrogels (114) der Hydrogelmatrix (110) übersteigt, wobei das Hydrogel des Sensormatrixmaterials (122) insbesondere einen Wassergehalt von mindestens 70 Gewichtsprozent, vorzugsweise von mindestens 85 Gewichtsprozent, aufweist.

9. Implantat (110) nach einem der vorhergehenden Ansprüche, wobei das Sensormatrixmaterial (122) mindestens eines der folgenden Materialien aufweist: ein Alginat, insbesondere ein ionisch vernetztes Alginat; ein Polysaccharid; ein Polysaccharidderivat, insbesondere Carboxymethylcellulose; ein synthetisches kovalent oder über Wasserstoffbrückenbindung vernetztes oder ionisch vernetztes Polymer oder Copolymer, insbesondere Polyvinylalkohol und/oder Polyhydroxyethylmethacrylat; ein Polyacrylamid; ein eine Acrylsäure-Einheit enthaltendes Copolymer; ein Acrylsäurederivat oder ein Methacrylsäurederivat, insbesondere Dimethylacrylamid, Hydroxyethyacrylat oder Methacrylsäure.

10. Implantat (110) nach einem der vorhergehenden Ansprüche, wobei die Hydrogelmatrix (110) und das Sensormatrixmaterial (122) ein chemisch identisches Hydrogel (114) mit jeweils unterschiedlichem Vernetzungsgrad aufweisen, wobei insbesondere das Hydrogel (114) der Hydrogelmatrix (110) einen höheren Vernetzungsgrad aufweist als das Hydrogel des Sensormatrixmaterials (122).

11. Implantat (110) nach einem der vorhergehenden Ansprüche, wobei die Referenzkomponente (132) mindestens eine lumineszierende Komponente, insbesondere eine Fluoreszenzkomponente, aufweist, wobei die Lumineszenzeigenschaften der luminszierenden Komponente zumindest weitgehend Analyt-invariant sind.

12. Implantat (110) nach einem der vorhergehenden Ansprüche, wobei die Referenzkomponente (132) an der Oberfläche und/oder im Inneren des Referenzmatrixmaterials (130) physikalisch und/oder chemisch gebunden ist.

13. Implantat (110) nach dem vorhergehenden Anspruch, wobei die physikalische und/oder chemische Bindung mindestens eine der folgenden Bindungen umfasst: eine kovalente Bindung; eine Komplexbindung; eine ionische Wechselwirkung und/oder eine ionische Bindung.

14. Implantat (110) nach einem der beiden vorhergehenden Ansprüche, wobei die Referenzkomponente (132) zumindest teilweise durch Polymerisation mit dem Referenzmatrixmaterial (130) verbunden ist.

15. Implantat (110) nach einem der drei vorhergehenden Ansprüche, wobei die Referenzkomponente (132) zumindest weitgehend physikalisch in dem Referenzmatrixmaterial (130) immobilisiert ist, insbesondere durch eine Quellung.

## Claims

1. Implant (110) for detecting at least one analyte (126) in a body fluid, in particular an eye fluid, the implant (110) being designed to be implanted in a body tissue of a patient, in particular a tissue layer and/or a chamber of an eye of the patient, the implant (110) having a hydrogel matrix (110) with at least one hydrogel (114), the implant (110) also having sensor particles (116), the sensor particles (116) having at least one sensor matrix (120) with a sensor matrix material (122) and at least one sensor material (124), **characterized in that** the implant (110) also has reference particles (128), the reference particles (128) having a reference matrix material (130) and an at least substantially analyte-invariant reference component (132) bonded thereto, and **in that** the sensor particles (116) and the reference particles (128) are dispersed homogeneously in the hydrogel matrix (110).

2. Implant (110) according to the preceding claim, the sensor material (124) having at least one of the following materials: concanavalin A; glucose oxidase; glucose dehydrogenase; hexokinase; glucose/galactose-binding protein; a protein and/or a fragment functionally equivalent to a protein; a mutant of hexokinase; a mutant of glucose/galactose-binding protein; a borate ester derivative.

3. Implant (110) according to one of the two preceding claims, the sensor particles (116) having a shell (118), in particular a membrane, at least partially permeable to the analyte (126).

4. Implant (110) according to the preceding claim, the shell (118) having diffusion-inhibiting properties for the at least one sensor material (124), in particular being at least substantially impermeable to the at least one sensor material (124).

5. Implant (110) according to one of the two preceding claims, the shell (118) having at least one of the following materials: a crosslinked protein; a polysaccharide; a crosslinked polysaccharide; a polymer, in particular a hydrogel with a density exceeding the density of the hydrogel of the hydrogel matrix (110); a layer-by-layer coating.

6. Implant (110) according to one of the preceding claims, the sensor material (124) having at least one of the following materials:
- a molecule and/or a group which, in the presence of an analyte (126), changes at least one chemical or physical property, in particular a luminescence property, in particular a fluorescence property;
- a molecule and/or a group where the analyte (126), on approaching and/or reacting with the molecule and/or the group, changes at least one chemical or physical property, in particular a luminescence property, in particular a fluorescence property;
- a molecule and or a group where the analyte (126) undergoes a chemical or physical bonding to the molecule and/or the group, which bonding can be detected by a change in at least one chemical or physical property, in particular by a change in a luminescence property, in particular by a change in a fluorescence property.

7. Implant (110) according to one of the preceding claims, the at least one sensor matrix material (122) and the hydrogel matrix (110) being designed in such a way that the at least one sensor material (124) has a higher diffusion coefficient in the sensor matrix material (122) than in the hydrogel matrix (HO), in particular than in the at least one hydrogel (114) of the hydrogel matrix (110), the sensor matrix material (122) preferably having a lower density than the hydrogel matrix (110), in particular than the hydrogel (114) of the hydrogel matrix (110).

8. Implant (110) according to one of the preceding claims, the sensor matrix material (122) having a hydrogel with a water content that exceeds the water content of the hydrogel (114) of the hydrogel matrix (110), the hydrogel of the sensor matrix material (122) having a water content of at least 70 percent by weight, preferably of at least 85 percent by weight.

9. Implant (110) according to one of the preceding claims, the sensor matrix material (122) having at least one of the following materials: an alginate, in particular an ionically crosslinked alginate; a polysaccharide; a polysaccharide derivative, in particular carboxymethylcellulose; a synthetic polymer or copolymer crosslinked covalently or via a hydrogen bond or an ionically crosslinked polymer or copolymer, in particular polyvinyl alcohol and/or polyhydroxyethyl methacrylate; a polyacrylamide; a copolymer containing an acrylic acid unit; an acrylic acid derivative or a methacrylic acid derivative, in particular dimethylacrylamide, hydroxyethyl acrylate or methacrylic acid.

10. Implant (110) according to one of the preceding claims, the hydrogel matrix (110) and the sensor matrix material (122) having a chemically identical hydrogel (114) with in each case a different degree of crosslinking, the hydrogel (114) of the hydrogel matrix (110) in particular having a higher degree of crosslinking than the hydrogel of the sensor matrix material (122).

11. Implant (110) according to one of the preceding claims, the reference component (132) having at least one luminescent component, in particular a fluorescence component, the luminescence properties of the luminescent component being at least substantially analyte-invariant.

12. Implant (110) according to one of the preceding claims, the reference component (132) being bonded physically and/or chemically on the surface and/or in the interior of the reference matrix material (130).

13. Implant (110) according to the preceding claim, the physical and/or chemical bond comprising at least one of the following bonds: a covalent bond; a complex bond; an ionic interaction and/or an ionic bond.

14. Implant (110) according to one of the two preceding claims, the reference component (132) being connected to the reference matrix material (130) at least partially by polymerization.

15. Implant (110) according to one of the three preceding claims, the reference component (132) being at least substantially immobilized physically in the reference matrix material (130), in particular by swelling.

## Revendications

1. Implant (110) pour la détection d'au moins un analyte (126) dans un liquide corporel, notamment un liquide oculaire, l'implant (110) étant conçu pour être implanté dans un tissu corporel d'un patient, notamment une couche tissulaire et/ou une chambre d'un fil du patient, l'implant (110) comprenant une matrice d'hydrogel (110) contenant au moins un hydrogel (114), l'implant (110) comprenant en outre des particules de détection (116), les particules de détection (116) comprenant au moins une matrice de détection (120) contenant un matériau de matrice de détection (122) et au moins un matériau de détection (124), **caractérisé en ce que** l'implant (110) comprend en outre des particules de référence (128), les particules de référence (128) comprenant un matériau de matrice de référence (130) et un composant de référence (132) relié à celui-ci et au moins essentiellement invariant avec l'analyte, et **en ce que** les particules de détection (116) et les particules de référence (128) sont dispersées sous forme homogène dans la matrice d'hydrogel (110).

2. Implant (110) selon la revendication précédente, dans lequel le matériau de détection (124) comprend au moins un des matériaux suivants : la concanavaline A ; une oxydase de glucose ; une déshydrogénase de glucose ; une hexokinase ; une protéine de liaison du glucose galactose ; une protéine et/ou un fragment fonctionnellement équivalent à une protéine ; un mutant d'hexokinase ; un mutant de protéine de liaison du glucose galactose ; un dérivé d'ester de l'acide borique.

3. Implant (110) selon l'une quelconque des deux revendications précédentes, dans lequel les particules de détection (116) comprennent une enveloppe (118) au moins partiellement perméable pour les analytes (126), notamment une membrane.

4. Implant (110) selon la revendication précédente, dans lequel l'enveloppe (118) présente des propriétés d'inhibition de la diffusion pour ledit au moins un matériau de détection (124), notamment est au moins essentiellement imperméable audit au moins un matériau de détection (124).

5. Implant (110) selon l'une quelconque des deux revendications précédentes, dans lequel l'enveloppe (118) comprend au moins un des matériaux suivants : une protéine réticulée ; un polysaccharide ; un polysaccharide réticulé ; un polymère, notamment un hydrogel ayant une densité dépassant la densité de l'hydrogel de la matrice d'hydrogel (110) ; une couche « layer by layer ».

6. Implant (110) selon l'une quelconque des revendications précédentes, dans lequel le matériau de détection (124) comprend au moins un des matériaux suivantes :
- une molécule et/ou un groupe, qui en présence d'un analyte (126) modifie au moins une propriété chimique ou physique, notamment une propriété de luminescence, notamment une propriété de fluorescence ;
- une molécule et/ou un groupe, l'analyte (126) modifiant lors d'un rapprochement et/ou d'une réaction avec la molécule et/ou le groupe au moins une propriété chimique ou physique, notamment une propriété de luminescence, notamment une propriété de fluorescence ;
- une molécule et/ou un groupe, l'analyte (126) formant une liaison chimique ou physique avec la molécule et/ou le groupe, qui est détectable par modification d'une propriété chimique ou physique, notamment par modification d'une propriété de luminescence, notamment par modification d'une propriété de fluorescence.

7. Implant (110) selon l'une quelconque des revendications précédentes, dans lequel ledit au moins un matériau de matrice de détection (122) et la matrice d'hydrogel (110) sont configurés de sorte que ledit au moins un matériau de détection (124) présente dans le matériau de matrice de détection (122) un coefficient de diffusion plus élevé que dans la matrice d'hydrogel (HO), notamment que dans ledit au moins un hydrogel (114) de la matrice d'hydrogel (110), le matériau de matrice de détection (122) présentant de préférence une densité plus faible que la matrice d'hydrogel (110), notamment que l'hydrogel (114) de la matrice d'hydrogel (110).

8. Implant (110) selon l'une quelconque des revendications précédentes, dans lequel le matériau de matrice de détection (122) comprend un hydrogel ayant une teneur en eau qui dépasse la teneur en eau de l'hydrogel (114) de la matrice d'hydrogel (110), l'hydrogel du matériau de matrice de détection (122) présentant notamment une teneur en eau d'au moins 70 pour cent en poids, de préférence d'au moins 85 pour cent en poids.

9. Implant (110) selon l'une quelconque des revendications précédente, dans lequel le matériau de matrice de détection (122) comprend au moins un des matériaux suivants : un alginate, notamment un alginate réticulé ioniquement ; un polysaccharide ; un dérivé de polysaccharide, notamment la carboxyméthylcellulose ; un polymère ou copolymère synthétique réticulé par des liaisons covalentes ou par des ponts hydrogène ou réticulé ioniquement, notamment l'alcool polyvinylique et/ou le polyméthacrylate d'hydroxyéthyle ; un polyacrylamide ; un copolymère contenant une unité acide acrylique ; un dérivé d'acide acrylique ou un dérivé d'acide méthacrylique, notamment le dimétylacrylamide, l'acrylate d'hydroxyéthyle ou l'acide méthacrylique.

10. Implant (110) selon l'une quelconque des revendications précédentes, dans lequel la matrice d'hydrogel (110) et la matrice de matériau de détection (122) comprennent un hydrogel chimiquement identique (114) présentant à chaque fois un degré de réticulation différent, l'hydrogel (114) de la matrice d'hydrogel (110) présentant notamment un degré de réticulation plus élevé que l'hydrogel du matériau de matrice de détection (122).

11. Implant (110) selon l'une quelconque des revendications précédentes, dans lequel le composant de référence (132) comprend au moins un composant luminescent, notamment un composant de fluorescence, les propriétés de luminescence du composant luminescent étant au moins essentiellement invariantes avec l'analyte.

12. Implant (110) selon l'une quelconque des revendications précédentes, dans lequel le composant de référence (132) est relié physiquement et/ou chimiquement à la surface et/ou à l'intérieur du matériau de matrice de référence (130).

13. Implant (110) selon la revendication précédente, dans lequel la liaison physique et/ou chimique comprend au moins une des liaisons suivantes : une liaison covalente ; une liaison de complexation ; une interaction ionique et/ou une liaison ionique.

14. Implant (110) selon l'une quelconque des deux revendications précédentes, dans lequel le composant de référence (132) est relié au moins en partie par polymérisation avec le matériau de matrice de référence (130) .

15. Implant (110) selon l'une quelconque des trois revendications précédentes, dans lequel le composant de référence (132) est immobilisé au moins essentiellement physiquement dans le matériau de matrice de référence (130), notamment par un gonflement.
